# EUROPÄISCHE PATENTSCHRIFT

(11) **EP 3 794 611 B1**
(45) Veröffentlichungstag und Bekanntmachung des Hinweises auf die Patenterteilung: **10.06.2026**
(21) Anmeldenummer: 19720565.1
(22) Anmeldetag: 29.04.2019
(51) Int. Cl.: G16H 50/20, A61B 5/00, A45D 44/00, G06N 3/0464, G06N 3/09, G16H 20/10

(54) **ERMITTELN VON ÄUSSEREN SCHÄDEN VON HAAREN**
DETERMINING EXTERNAL HAIR DAMAGE
DÉTERMINATION DES DOMMAGES EXTÉRIEURS DES CHEVEUX

(30) Priorität: 16.05.2018 DE 102018207558
(43) Veröffentlichungstag der Anmeldung: 24.03.2021
(73) Patentinhaber: Henkel AG & Co. KGaA, 40589 Düsseldorf (DE)
(72) Erfinder: KNUEBEL, Hans Georg, 40219 Düsseldorf (DE); SCHULZE ZUR WIESCHE, Erik, 22453 Hamburg (DE)
(86) Internationale Anmeldenummer: PCT/EP2019/060853
(87) Internationale Veröffentlichungsnummer: WO 2019/219355

(56) Entgegenhaltungen:
- DE-A1- 102016 208 631
- DE-A1- 102016 225 674
- JP-B2- 4 071 891
- US-A1- 2017 038 297
- EGMONT-PETERSEN M ET AL: "Image processing with neural networks-a review", PATTERN RECOGNIT, ELSEVIER, GB, vol. 35, no. 10, 19 June 2002 (2002-06-19), pages 2279 - 2301, XP004366785, ISSN: 0031-3203, DOI: 10.1016/S0031-3203(01)00178-9
- ANONYMOUS: "Pattern recognition - Wikipedia, the free encyclopedia", 4 November 2015 (2015-11-04), XP055253978, Retrieved from the Internet <URL:https://en.wikipedia.org/w/index.php?title=Pattern_recognition&oldid=688980762> [retrieved on 20160229]

## Beschreibung

Die Erfindung betrifft eine Anordnung zum Bestimmen von äußeren Schäden von Haaren, ein Computerprogrammprodukt, welches ausgeführt ist, auf einer Recheneinheit ausgeführt zu werden, und ein Verfahren zum Bestimmen von äußeren Schäden von Haaren.

Bei einer Behandlung von Haar mit kosmetischen Produkten kann eine Wirkung des Produkts, z.B. eine Intensität einer Färbung, stark von einer Schädigung des Haars abhängen. Die Schädigung oder der Schaden kann insbesondere eine Schadensart (welcher Schaden) und einen Schadensgrad (Umfang des Schadens) bezeichnen, sich aber auch auf mehrere verschiedene Schadensarten und zugehörige Schadensgrade beziehen. Geschädigtes Haar ist oftmals schwer handhabbar und glanzlos.

Es gibt viele verschiedene Haarbehandlungsmittel am Markt, welche unterschiedliche Haareigenschaften oder -parameter, wie beispielsweise den Glanz, verbessern sollen. In vielen Fällen weiß der Anwender solcher Produkte aber nicht, wie stark (Schadensgrad) und auf welche Weise (Schadensart) seine Haare geschädigt sind. Dies kann dazu führen, dass der Anwender zu in seinem speziellen Fall weniger geeigneten Produkten greift und nach ihrer Anwendung mit deren Wirksamkeit unzufrieden ist.

Deshalb kann eine Ermittlung einer Schädigung des Haars von großer Bedeutung sein.

Haar kann durch natürliche oder künstlich herbeigeführte Vorgänge geschädigt werden. Die natürlichen Vorgänge können beispielsweise eine kombinierte (z.B. gleichzeitige) Einwirkung von UV-Licht und Sauerstoff (O2) auf das Haar aufweisen. Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle umfassen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der Schädigungsvorgang kann dabei ursächlich durch eine Oxidation von Aminosäuren, beispielsweise eine Oxidation der im Haar sehr häufig vorkommenden Aminosäuren Cystin und Cystein zu Cysteinsäure, erfolgen. Cystin kann im Haar intermolekulare Disulfidbrücken (auch als S-S-Brücken bezeichnet) ausbilden, so dass das Cystin äußerst wichtig ist für die mechanische Stabilität des Haars.

Die Oxidation dieser Brücken zu Cysteinsäure kann die mechanische Stabilität des Haars zerstören und bei mehrfachen Anwendungen sogar zu einem vollständigen Haarbruch führen.

Allerdings können bereits vorher makroskopisch wahrnehmbare, z.B. fühlbare, Eigenschaften des Haars, beispielsweise eine Oberflächenrauigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar.

Immer mehr Anwender von Produkten wünschen sich ein auf ihre individuellen Bedürfnisse abgestimmtes Produkt. Dies gilt insbesondere auch für Schönheitsprodukte wie Haut- und/oder Haarbehandlungsmittel.

DE 102016225674 A1 beschreibt ein Verfahren zum Ermitteln eines Schädigungsgrads von Haar, das aufweist: während eines Belichtens einer Haarprobe des Haars mit Licht, Registrieren von Licht, welches von der Haarprobe abgestrahlt wird, Ermitteln, basierend auf dem registrierten Licht, von ersten Bereichen der Haarprobe, die das Licht mit höherer Interferenz reflektieren und zweiten Bereichen der Haarprobe, die das Licht mit niedrigerer Interferenz reflektieren und Ermitteln eines Schädigungsgrads der Haarprobe basierend auf den Größen der ersten Bereiche und der zweiten Bereiche.

DE 102016208631 A1 beschreibt ein Verfahren zum Ermitteln eines Schädigungsgrads von Haar. Das Verfahren kann aufweisen: während eines Belichtens einer Haarprobe mit UV-Licht (beispielsweise mittels einer UV-LED), Registrieren von Fluoreszenzlicht, welches von der Haarprobe emittiert wird, Ermitteln einer Fluoreszenzintensität anhand des registrierten Lichts, und Ermitteln des Schädigungsgrads des Haars unter Einbeziehung der Fluoreszenzintensität.

US 20170038297 A1 beschreibt eine Vorrichtung und ein Verfahren zum kundenspezifischen Färben von Haaren, das umfasst:
a) Durchführen einer Vielzahl von Lichtstreuungsmessungen an einer Haarprobe, so dass für jede Lichtstreuungsmessung die Haarprobe aus einer jeweils anderen Richtung beleuchtet wird;
b) Vergleichen der Ergebnisse der Lichtstreuungsmessungen;
c) Berechnen eines anfänglichen Schadenszustands des Haars der Probe in Übereinstimmung mit den Ergebnissen des Vergleichs durch Vergleichen der Ergebnisse der Lichtstreuungsmessungen;
d) Ermitteln eines anfänglichen Farbzustands des Haars der Probe;
e) Berechnen einer Haarfärbezusammensetzung, von der vorhergesagt wird, dass sie die Haarprobe von dem anfänglichen Farbzustand in einen Zielfarbzustand umwandelt, so dass als Reaktion auf die Bestimmung eines größeren oder geringeren Ausmaßes der anfänglichen Schädigung eine Konzentration von künstlichem(n) Farbstoff(en) innerhalb der berechneten Färbezusammensetzung reduziert oder erhöht wird.

JP 4071891 B2 beschreibt ein System, das einem Kunden Empfehlungen für Haarprodukte gibt, die auf den Ergebnissen einer Haardiagnose beruhen. Dazu wird eine umfassende Haardiagnose durchgeführt, die auf der Textur der Haare und der Analyse von Schuppenschicht, Kortex und Mark beruht. Zusätzlich zu einem Bild des Haares mit einem Mikroskop werden der Glanz und der Farbton des Haares aufgenommen und der Zustand des Marks, der Schuppenschicht und des Kortex des Haares auf der Grundlage aller Daten beobachtet. Außerdem werden die Daten der mündlichen Untersuchung kombiniert, um eine umfassende Bewertung vorzunehmen. Darüber hinaus wird eine Datenbank zur Verfügung gestellt, in der frühere Beobachtungsdaten und andere nützliche Daten für die Bewertung des Haares gesammelt werden.

Es kann als Aufgabe der vorliegenden Erfindung betrachtet werden, das Bestimmen von äußeren Schäden von Haaren auf einfache Weise zu ermöglichen und eine darauf abgestimmte Haarbehandlungsanweisung auszugeben.

Diese Aufgabe wird gelöst mit den Merkmalen der unabhängigen Ansprüche. Weiterbildungen der Erfindung ergeben sich aus den abhängigen Ansprüchen und aus der folgenden Beschreibung.

Der Erfindung liegen unter anderem die folgenden Erkenntnisse zu Grunde: Die Entwicklung personalisierter haarkosmetischer Produkte wird ermöglicht durch eine umfassende Charakterisierung des Haarzustandes einer Person, damit eine effektive individualisierte Produktempfehlung, z. B. für Haarpflegeprodukte, durchgeführt werden kann. Die Charakterisierung kann grundsätzlich durch die Person selbst erfolgen, im Friseursalon durch einen Friseur oder an einer Verkaufsstelle (Point of Sale) von Haarbehandlungsmitteln. Für alle Varianten sind dabei einfache, preiswerte und robuste Verfahren hilfreich und teilweise erforderlich. Die Bestimmung des äußeren und inneren Haarschadens ist dabei prinzipiell mit einer Vielzahl von biophysikalischen Messmethoden möglich. Viele dieser Verfahren sind jedoch apparativ ausgesprochen aufwändig und für Endanwender nicht nutzbar. Insbesondere alle mechanischen Verfahren sind sehr anspruchsvoll, damit die relevanten mechanischen Daten mit der notwendigen Präzision ermittelt werden können. Darüber hinaus ist bei diesen Verfahren ein beträchtlicher Kalibrier-, Wartungs- und Reinigungsaufwand gegeben. Damit besteht ein großer Bedarf an nichtmechanischen, idealerweise berührungslosen Verfahren zur Haaranalyse.

Es wurde nun gefunden, dass sich die Fragestellung, ob ein Haar oder eine Haarprobe geschädigt oder ungeschädigt ist (eine weitere Unterteilung auf einer Skala mit Schädigungsgraden mit mehr als zwei Stufen ist ebenfalls möglich) ohne größeren Aufwand mit Hilfe von Verfahren des maschinengestützten Lernens, beispielsweise unter Nutzung von Techniken des "Deep Learnings" beantworten lässt. Insbesondere ermöglicht dies eine isolierte Quantifizierung des morphologischen Schadens an der Oberfläche eines Haares, ohne dass die Eigenschaften des gesamten Haares erfasst werden.

Gemäß einem Aspekt ist eine Anordnung zum Bestimmen von äußeren Schäden von Haaren angegeben. Die Anordnung weist eine Leseeinheit und eine Verarbeitungseinheit auf. Die Leseeinheit ist ausgeführt, optische Merkmale von Haaren zu erhalten. Die Verarbeitungseinheit ist ausgeführt, die optischen Merkmale von der Leseeinheit zu erhalten und aus den optischen Merkmalen Oberflächeneigenschaften der Haare zu ermitteln. Die Verarbeitungseinheit implementiert einen Mustererkennungsalgorithmus und ist ausgeführt, mittels des Mustererkennungsalgorithmus die Oberflächeneigenschaften einem vorbestimmten Schaden enthaltend mindestens eine Schadensart und einen zugehörigen Schadensgrad zuzuordnen und den Schaden auszugeben.

Als optische Merkmale von Haaren werden optisch erfassbare Merkmale verstanden, beispielsweise solche Merkmale, welche in einer Bildaufnahme im sichtbaren Lichtspektrum zu sehen sind. Insbesondere betreffen die optischen Merkmale Oberflächenmerkmale, beispielsweise die Struktur der Cuticula, welche die äußere Schicht eines Haars ist und auch als Schuppenschicht bezeichnet werden kann. Wird in der weiteren Beschreibung auf die Haaroberfläche Bezug genommen, so ist dies gleichbedeutend mit der äußeren Oberfläche der Cuticula.

Die Leseeinheit erhält die optischen Merkmale beispielsweise von einer optischen Erfassungseinheit oder aus einem Datenspeicher oder ruft die optischen Merkmale in Form von Bildinformation von der optischen Erfassungseinheit oder dem Datenspeicher ab.

Die Leseeinheit kann als Zugriffsschnittstelle ausgestaltet sein, welche eine Bildquelle (optische Erfassungseinheit oder Datenspeicher) anschließt oder die Bildquelle kommunikativ mit der Verarbeitungseinheit koppelt.

Die Verarbeitungseinheit ermittelt basierend auf den optischen Merkmalen oder Bildern von Haaren die Oberflächeneigenschaften der Haare. Die Verarbeitungseinheit kann zu diesem Zweck einen Bildverarbeitungsalgorithmus implementieren, welcher aus einem Linienverlauf in einem Bild der Haaroberfläche die Struktur der Haaroberfläche extrahiert. Beispielsweise kann die Verarbeitungseinheit die Form einzelner Cuticula-Zellen oder Schuppen ermitteln. Einzelne Cuticula-Zellen werden in einer Bildaufnahme anhand eines Linienverlaufs auf der Haaroberfläche erkannt.

Die Verarbeitungseinheit wendet einen Mustererkennungsalgorithmus an, um die Form der Cuticula-Zellen zu erfassen und daraus einen äußeren Schaden eines Haares zu ermitteln, indem beispielsweise die erkannten Oberflächeneigenschaften mit vorab bekannten und hinterlegen Schäden an Haaren abgeglichen und einem Schaden zugeordnet werden.

Ein Schaden kann sich auf eine Schadensart und einen zugehörigen Schadensumfang oder Schadensgrad beziehen. Als Schadensart kommen unter anderem die folgenden Aspekte in Frage: Form der Ränder der Cuticula-Zellen (rund, ausgefranst), abstehende Cuticula, beispielsweise an den Rändern von dem Cortex des Haares gelöste oder abstehende Cuticula-Zellen, Länge der exponierten Cuticula-Zellen, Abstände der Kanten der Cuticula-Zellen, Löcher und/oder Blasen in der Oberfläche oder einzelnen Cuticula-Zellen, Oberflächenrauigkeit (z.B. Erhebungen und/oder Vertiefungen in der Oberfläche), Ablagerungen auf der Cuticula, Sichtbarkeit der Endocuticula.

Zu jeder dieser Schadensarten kann ein Schadensumfang oder Schadensgrad angegeben werden.

Verschiedene Formen von Rändern der Cuticula-Zellen können einem Schadensgrad zugewiesen werden, so dass eine Bildaufnahme eines Haares dem optisch ähnlichsten Schadensgrad zugeordnet wird. So kann auf einfache Weise der Schadensgrad betreffend die Ränder zugeordnet werden.

Allgemein kann ein äußerer Schaden im Sinne dieser Beschreibung eine Beschädigung der Cuticula von Haar mit einer Schadenstiefe von 1 bis 5 µm sein. Die Beschädigung kann eine Veränderung des Erscheinungsbildes der Oberfläche der Cuticula sein und insbesondere Form, Art, Länge, Anzahl und/oder Abstand von Vertiefungen in der Cuticula betreffen.

Der gleiche Ansatz wird für die übrigen Schadensarten angewandt. Die Verarbeitungseinheit gleicht die Oberflächeneigenschaften eines untersuchten Haares mit vorbestimmten Schäden ab und ermittelt basierend auf diesem Abgleich den Schaden der Haarprobe.

In einem nicht beanspruchten Beispiel kann der Mustererkennungsalgorithmus aber nicht nur über einen Bildabgleich und Ähnlichkeitsermittlung erfolgen. Beispielsweise kann für die Mustererkennung auch ein künstliches neuronales Netzwerk genutzt werden, welches den Schaden nicht durch Abgleich mit bekannten Aufnahmen ermittelt, sondern basierend auf der Konfiguration des künstlichen neuronalen Netzwerks. Hierzu kann das neuronale Netzwerk eine Konfigurationsphase durchlaufen, in welcher ihm verschiedene Bildaufnahmen von Haarproben jeweils mit einem festgelegten oder vorgegebenen Schaden zugeführt werden. So lernt das neuronale Netzwerk, aus Bildaufnahmen einen Schaden zu erkennen, ohne konkret einen Abgleich einer Haarprobenbildaufnahme mit anderen Bildaufnahmen vorzunehmen.

Durch die Mustererkennung kann einer Haarprobe einer Schadenskategorie zugeordnet werden.

Schadenskategorien können ein numerischer Wert sein, welcher anzeigt, wie stark das Haar äußerlich beschädigt ist und/oder welcher Art die Beschädigung ist. Der numerische Wert kann mehrere Ziffern enthalten, wovon die Position der Schadensart zugeordnet ist und der Wert der Ziffer den Schadensgrad anzeigt. Beispielsweise könnte die Zahl "0-2-1-3-4-2" aussagen, dass die Form der Ränder der Cuticula-Zellen (erste Ziffer) unauffällig ist oder auf keinen Schaden hinweist (Wert 0), die Cuticula-Zellen an den Rändern von dem Cortex des Haares gelöst sind (zweite Ziffer, Wert 2), die Länge der exponierten Cuticula-Zellen auf geringe Schäden hinweist (dritte Ziffer, Wert 1), die Abstände der Kanten der Cuticula-Zellen (vierte Ziffer) auf einen hohen Schadensgrad hinweise (Wert 3), Löcher und/oder Blasen in der Oberfläche oder einzelnen Cuticula-Zellen (fünfte Ziffer) stark vorhanden sind (Wert 4), und Ablagerungen auf der Cuticula sowie Sichtbarkeit der Endocuticula (sechste Ziffer) ebenfalls leicht vorhanden sind (Wert 2). Jede einzelne Ziffer kann bis zu zehn unterschiedliche Schadensgrade anzeigen, nämlich 0 bis 9. Es ist auch denkbar, die Skala weiter zu unterteilen und für jede Schadensart zweistellige oder mehrstellige Zahlen zu verwenden. Allerdings liegen die Grenzen einer solchen Einteilung der Skala in der Frage, wie genau die Schäden zu erkennen sind und wie sinnvoll eine Unterteilung in viele Grade ist. Typischerweise kann eine Unterteilung in zwei bis vier Schadensgrade ausreichend sein, um zu unterscheiden ob keine Beschädigung, leichte Beschädigung, überdurchschnittliche Beschädigung, oder starke Beschädigung vorliegt.

Die Schadensart und der Schadensgrad wird nicht einzig basierend auf einer Bildaufnahme bestimmt, sondern durch Rückgriff auf bekannte Haarproben mit zugeordnetem Schaden, entweder durch einen Ähnlichkeitsvergleich der Haarprobe mit den bereits klassifizierten Bildaufnahmen oder durch Zuführen der Bildaufnahme der Haarprobe an ein künstliches neuronales Netzwerk, welches der Haarprobe einen Schaden basierend auf seiner Konfiguration zuordnet.

Hierdurch kann ein morphologischer Schaden an der Oberfläche eines Haares bestimmt werden, ohne irgendwelche Aussagen über die innere Beschaffenheit des Haares zu treffen. Für gewisse Behandlungen des Haares mag die innere Beschaffenheit des Haares irrelevant oder vernachlässigbar sein. Verfahren, welche die innere Beschaffenheit erfassen, mögen also für manche Behandlungen einen irreführenden Wert für die Schädigung des Haares angeben. Dies wird vorliegend verhindert, indem die bloße äußere morphologische Beschaffenheit herangezogen wird.

Eine Bildaufnahme einer Haarprobe enthält bevorzugt ein einzelnes Haar. Es können von einer Person auch mehrere Bildaufnahmen von unterschiedlichen Haaren angefertigt werden, um eine aussagekräftige Aussage über den durchschnittlichen Zustand der Haare zu erhalten. Eine Bildaufnahme eines Haares zeigt typischerweise die gesamte Dicke oder den gesamten Durchmesser eines Haares und erstreckt sich über einen Teilabschnitt der Länge des Haares. Der in der Bildaufnahme enthaltene Längsabschnitt des Haares kann in etwa der Dicke des Haares entsprechen, also zwischen wenigen Hundertstel Millimetern bis hin zu wenigen Zehntel Millimetern, z.B. zwischen 0,04 mm und 0,15 mm, liegen.

Die Bildaufnahme der Haarprobe liegt bevorzugt in der gleichen Auflösung vor, wie die Bildaufnahmen, mit denen der Mustererkennungsalgorithmus konfiguriert wurde. Bevorzugt werden die Bildaufnahmen von allen Haarproben in der gleichen Auflösung angefertigt und der Verarbeitungseinheit zugeführt.

Gemäß einer nicht beanspruchten Ausführungsform ist der Mustererkennungsalgorithmus ein künstliches neuronales Netzwerk, welches eine Eingangsschicht, mindestens eine verborgene Schicht und eine Ausgangsschicht aufweist, wobei das neuronale Netzwerk ausgeführt ist, den Schaden basierend auf den Oberflächeneigenschaften auszugeben.

Es kann insbesondere vorgesehen sein, die Ansätze von "deep learning" auf das künstliche neuronale Netz anzuwenden. Das neuronale Netz kann eine hohe Tiefe haben, was mit einer hohen Anzahl an verborgenen Schichten einhergeht. Jede Schicht weist eine Mehrzahl von sogenannten künstlichen Neuronen auf.

Das neuronale Netz ist so konfiguriert, dass eine Bildaufnahme einer Haarprobe der Eingangsschicht zugeführt wird, die Neuronen der Eingangsschicht mit den Neuronen der verborgenen Schichten interagieren, wobei verschiedene verborgene Schichten miteinander interagieren, und die Ausgangsschicht den Schaden der Haarprobe anzeigt.

Gemäß einer weiteren Ausführungsform ist die Verarbeitungseinheit ausgeführt, den Mustererkennungsalgorithmus in einem Konfigurationsmodus zu betreiben, wobei die Verarbeitungseinheit ausgeführt ist, dem Mustererkennungsalgorithmus in dem Konfigurationsmodus optische Merkmale einer Vielzahl von Haaren mit unterschiedlichem Schaden zuzuführen und für jedes einzelne Haar sowie seine optischen Merkmale einen Schaden abzufragen und zuzuordnen.

Dieser Konfigurationsmodus kann auch als Lernmodus oder Trainingsmodus des neuronalen Netzes bezeichnet werden. Hierbei wird das neuronale Netz mit verschiedenen Schadenarten und Schadengraden vertraut gemacht und die Neuronen werden durch das neuronale Netz entsprechend miteinander vernetzt, um in der Lage zu sein, zu einer neuen und unbekannten Bildaufnahme einer Haarprobe den Schaden zu ermitteln und auszugeben.

Gemäß einer weiteren Ausführungsform ist die Leseeinheit ausgeführt, die optischen Merkmale von einer optischen Erfassungseinheit zu erhalten.

Die Leseeinheit kann unmittelbar mit einer optischen Erfassungseinheit kommunikativ gekoppelt sein. Die optische Erfassungseinheit kann eine Kamera oder eine anderweitige Anordnung zum Erstellen eines Abbildes einer Haarprobe sein.

Gemäß einer weiteren Ausführungsform ist die optische Erfassungseinheit ein Mikroskop, welches eine mindestens 100-fache Vergrößerung aufweist, bevorzugt eine mindestens 500-fache Vergrößerung, weiter bevorzugt eine mindestens 1000-fache Vergrößerung und noch weiter bevorzugt eine mindestens 2000-fache Vergrößerung.

Ausgehend von einem Haardurchmesser zwischen etwa 0,04 mm und 0,12 mm wird bei 1000-facher Vergrößerung ein Haar auf eine in der Bildaufnahme dargestellte Dicke von etwa 40 mm bis 120 mm vergrößert, was es erlaubt, einzelne Cuticula-Zellen oder Schuppen sowie deren Ränder zu erkennen.

Gemäß einer weiteren Ausführungsform ist die Leseeinheit ausgeführt, die optischen Merkmale aus Bildaufnahmen von Haaren zu erhalten.

Aus den in den Bildaufnahmen enthaltenen Linien kann eine Struktur der Haaroberfläche ermittelt werden. Diese Struktur ist die Grundlage für das Zuordnen eines Schadens. Die Bildaufnahmen können in einem digitalen Datenformat vorliegen.

Gemäß einer weiteren Ausführungsform ist die Leseeinheit ausgeführt, die Bildaufnahmen aus einem digitalen Datenspeicher auszulesen.

Beispielsweise werden Bilder von einer Haarprobe auf einem portablen digitalen Datenspeichermedium wie einer SD-Karte, einem USB-Speichermedium, einer Diskette, einer CD-ROM oder einer DVD gespeichert und können somit an eine entfernte Stelle verschickt werden, wo die Bildaufnahmen der Analyse durch die Anordnung zugeführt werden. Alternativ können die Bildaufnahmen auf einer Festplatte in einem Computer gespeichert und für die weitere Verarbeitung bereitgestellt werden.

Es ist möglich, dass die Bilder über eine Datenübertragungsstrecke an die Verarbeitungseinheit übertragen werden, und zwar ausgehend entweder unmittelbar von der optischen Erfassungseinheit an die Leseeinheit oder an die Verarbeitungseinheit oder ausgehend von dem Datenspeicher oder dem Computer, auf dem Bildaufnahmen gespeichert sind.

Die optische Erfassungseinheit kann eine Schnittstelle aufweisen, über welche eine Verbindung mit der Leseeinheit oder der Verarbeitungseinheit hergestellt wird. Diese Verbindung ist ausgeführt, Informationen in mindestens eine Richtung zu übertragen, wobei eine bidirektionale Kommunikation auch möglich ist. Bilddaten werden von der optischen Erfassungseinheit an die Verarbeitungseinheit übertragen und beispielsweise Steuerkommandos von der Verarbeitungseinheit an die optische Erfassungseinheit. Die Verbindung kann drahtgebunden oder drahtlos sein. Drahtgebundene Verbindungen können beispielsweise optische oder elektrische Signale für die Informationsübertragung nutzen. Drahtlose Verbindungen nutzen typischerweise elektromagnetische Wellen für die Signalübertragung, z.B. Funksignale oder auch optische Signale.

Für die Anbindung der optischen Erfassungseinheit an die Leseeinheit oder Recheneinheit können Protokolle verwendet werden, die gemäß den Grundsätzen vermaschter Netze (mesh network) arbeiten. Beispielsweise kann für die Datenübertragung und für die Anbindung der optischen Erfassungseinheit an die Leseeinheit oder Verarbeitungseinheit das Thread-Protokoll verwendet werden, welches auf IPv6 aufsetzt. Das Thread-Protokoll wird insbesondere eingesetzt, um automatisierte oder teilautomatisierte Geräte miteinander zu verbinden, in diesem Fall beispielsweise die optische Erfassungseinheit mit der Leseeinheit oder Verarbeitungseinheit.

Die optische Erfassungseinheit und die übrigen Elemente der Anordnung können in dem gleichen Raum oder räumlich voneinander getrennt (beispielsweise in getrennten Gebäuden) angeordnet sein. Für den Fall, dass die Bilddaten von der Erfassungseinheit an die Leseeinheit oder die Verarbeitungseinheit übertragen werden, ist lediglich eine Datenübertragungsstrecke zwischen diesen Elementen erforderlich, wie oben beschrieben.

Gemäß einer weiteren Ausführungsform weist die Anordnung eine Auswerteeinheit auf, wobei die Auswerteeinheit ausgeführt ist, Merkmale von Behandlungsmitteln zur Behandlung von Haaren mit dem Schaden einer untersuchten Haarprobe abzugleichen und eine Wirkung der Behandlungsmittel auf die Haare unter Berücksichtigung des ermittelten Schadens zu bestimmen.

Bei der Behandlung von Haaren handelt es sich selbstverständlich um eine nicht-therapeutische Behandlung.

Das Behandlungsmittel wird also in Abhängigkeit des ermittelten Schadens ausgesucht und von der Auswerteeinheit ausgegeben.

Gemäß einer weiteren Ausführungsform ist die Auswerteeinheit ausgeführt, von einem Behandlungsmitteldatenspeicher Hinweise zur Behandlung von Haaren gemäß dem ermittelten Schaden zu erhalten und die erhaltenen Hinweise auszugeben.

Die Auswerteeinheit kann als Prozessor oder Computer ausgestaltet sein. Ebenfalls ist denkbar, dass die Auswerteeinheit ein Softwaremodul ist, welches auf einem Prozessor läuft oder davon ausgeführt wird, und die oben genannten Funktionen ausführt. Das Softwaremodul der Auswerteeinheit kann auf demselben oder einem anderen Prozessor ausgeführt werden wie die Funktionen der Verarbeitungseinheit.

Die Auswerteeinheit kann die erhaltenen Hinweise an eine optische oder akustische Ausgabeeinheit weitergeben, damit die Hinweise von der Ausgabeeinheit einem menschlichen Benutzer oder Bediener bereitgestellt werden. Die akustische Ausgabeeinheit kann ein Lautsprecher sein, die optische Ausgabeeinheit kann ein Monitor oder ein Display sein.

Bei diesen Hinweisen zur Behandlung von Haaren kann es sich um allgemeine Hinweise (ohne Bezug zu einem bestimmten Behandlungsmittel) betreffend die Behandlung von Haaren handeln, es kann sich aber auch um Hinweise mit Bezug zu einem bestimmten Behandlungsmittel handeln. Die Hinweise können auch Erklärungen beinhalten, welche Verhaltensweisen welche Eigenschaften der Haare wie beeinflussen.

In dem Behandlungsmitteldatenspeicher können Informationen aus Studien sowie Informationen aus Literaturquellen und/oder wissenschaftlichen Veröffentlichungen enthalten sein. Die Auswerteeinheit kann ausgeführt sein, einem Nutzer in Abhängigkeit der erfassten Eigenschaften der Haare einen Auszug aus diesen Informationen auszugeben oder zumindest darauf hinzuweisen.

Gemäß einer weiteren Ausführungsform ist die Auswerteeinheit ausgeführt, Informationen von einem Nutzer abzufragen und diese Informationen beim Abfragen des Behandlungsmitteldatenspeichers zusätzlich zu berücksichtigen, um von dem Behandlungsmitteldatenspeicher Merkmale von Behandlungsmitteln zur Behandlung von Haaren gemäß den abgefragten Informationen zu erhalten.

Die abgefragten Informationen können mittels eines vorgegebenen Fragebogens erfasst werden, bei welchem einer Aussage durch den Nutzer mehr oder weniger Gewicht beigemessen wird oder auch aus einer von mehreren möglichen Antworten ausgewählt wird. Der vorgegebene Fragebogen kann sich insbesondere mit den Lebensgewohnheiten und außergewöhnlichen Belastungen des Nutzers befassen, zum Beispiel Ernährungsgewohnheiten, Dauer und Qualität des Schlafs, Trinkmenge, Art der Getränke, Verwendung von Genussmitteln (beispielsweise Nikotin, Alkohol), beruflichen Aktivitäten und Freizeitaktivitäten (viel Zeit außerhalb von Gebäuden bei jeglicher Witterung, Aufenthalt in den Bergen, Besuch eines Solariums). Ebenso können auch Alter, Geschlecht und Ethnizität des Benutzers abgefragt sowie für das Abfragen des Behandlungsmitteldatenspeichers herangezogen werden. Auch können die abgefragten Informationen sich auf eine gewünschte oder zu erreichende Eigenschaft der Haare beziehen und somit einen Sollzustand oder Wunschzustand angeben.

Die Informationen können über eine Eingabeeinheit eingegeben werden. Die Eingabeeinheit kann mit der Auswerteeinheit signaltechnisch verbunden sein. Die Eingabe kann beispielsweise über einen Touchscreen erfolgen, wobei dem Nutzer Aussagen oder Fragen präsentiert werden, welchen eine Antwort des Nutzers zugeordnet wird.

Die von dem Nutzer gelieferten Aussagen können der Verarbeitungseinheit aber auch auf anderem Wege verfügbar gemacht werden. Beispielsweise können diese Nutzerinformationen zusammen mit den Bildern der Haarprobe übermittelt werden.

Die Auswerteeinheit kann ein Nutzerprofil anlegen und die Haarprobe sowie die zugehörigen Aussagen oder Angaben des Nutzers speichern. Dies ermöglicht es, spätere Haarproben mit früheren Haarproben abzugleichen und eine Veränderung der Schäden über die Zeit zu beobachten und auszuwerten.

Gemäß einer weiteren Ausführungsform ist die Auswerteeinheit ausgeführt, Informationen über ein Behandlungsmittel, z.B. eine Produktbezeichnung, Informationen betreffend Inhaltsstoffe und/oder Zusammensetzung eines Behandlungsmittels und/oder Anwendungshinweise zur Behandlung von Haaren auszugeben.

Somit wird es einem Nutzer ermöglicht, sich selbst ein Bild über ein Behandlungsmittel in seiner Gesamtheit zu machen. Außerdem können dem Nutzer Anwendungshinweise bezogen auf ein Behandlungsmittel oder unabhängig hiervon gegeben werden. Die Anwendungshinweise können sich auf gewünschtes und/oder ungewünschtes Verhalten beziehen.

Gemäß einer weiteren Ausführungsform ist die Auswerteeinheit ausgeführt, nach dem Ausgeben von Merkmalen eines Behandlungsmittels und/oder Anwendungshinweisen eine Eingabe von einem Nutzer entgegen zu nehmen und basierend auf dieser Eingabe eine Aktion betreffend das ausgegebene Behandlungsmittel und/oder die ausgegebenen Anwendungshinweise zu veranlassen.

Die Aktion kann sich beispielsweise darauf beziehen, dass dem Nutzer ein Behandlungsmittel zum Kauf angeboten wird und der Nutzer über eine Eingabe den Kauf in die Wege leiten kann. Neben dem Kauf von Behandlungsmitteln können dem Nutzer auch weitergehende Informationen zum käuflichen Erwerb angeboten werden. Diese weitergehenden Informationen können sich auf detailliertere Behandlungs- und Anwendungshinweise beziehen. Das Programm empfängt beispielsweise die Anfrage, dass der Nutzer das Behandlungsmittel erwerben möchte, speichert die Anfrage und/oder übermittelt die Anfrage an ein Handelsunternehmen, welches die Behandlungsmittel vertreibt. Der Nutzer wird durch das Computerprogramm aufgefordert, seine persönlichen Daten (Adresse, Bankinformationen, Versendungspräferenz, etc.) über die Eingabeeinheit einzugeben.

Alternativ kann dem Benutzer ausgegeben werden, wo, beispielsweise in einem Drogeriemarkt, in einem Friseursalon, in einer Apotheke, etc. in seiner Nähe, er das ausgegebene Behandlungsmittel vor Ort erwerben kann.

Immer mehr Kunden wünschen sich ein individuell auf ihre Bedürfnisse abgestimmtes Produkt. Entsprechend kann dem Nutzer ein individuell für ihn hergestelltes Behandlungsprodukt empfohlen und ein Bestellvorgang, beispielsweise durch Aufrufen einer Internetseite eines Herstellers von individuellen Haarbehandlungsmitteln, eingeleitet werden.

Dabei kann es sich um ein speziell für den einen Nutzer hergestelltes Behandlungsmittel oder ein sogenanntes "mass customized" Produkt handeln. Bei einem "mass customized" Produkt kann durch Variation von wenigen, aus Kundensicht jedoch entscheidenden, Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurators abläuft. Dieser Konfigurator hilft dem Nutzer bei der Auswahl der Merkmale/Inhaltsstoffe und weist ihn auf die zulässigen/unzulässigen Merkmalskombinationen hin, wobei letztere dann nicht ausgewählt werden können.

Bei Haarbehandlungsmitteln umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Schädigungsgrad/Dehnungsgrad/etc. ungeeigneter Inhaltsstoffe vermieden werden. Umgekehrt kann die Auswahl für den ermittelten Schädigungsgrad/Dehnungsgrad/etc. geeigneter Inhaltsstoffe durch den Produktkonfigurator vorgegeben oder vorgeschlagen werden.

Ebenso ist es möglich, ein individuelles Haarbehandlungsmittel vor Ort, das heißt zum Beispiel in einem Friseursalon oder an einem Verkaufspunkt von Haarbehandlungsmitteln, wie beispielsweise ein Drogeriemarkt, mittels einer Mischvorrichtung, vorzugsweise einer intelligenten Mischvorrichtung (Smart Mixer), herzustellen.

Gemäß einem weiteren Aspekt ist ein Computerprogrammprodukt angegeben, welches ausgeführt ist, auf einer Recheneinheit ausgeführt zu werden. Das Computerprogrammprodukt ist ausgeführt, einen Prozessor der Recheneinheit anzuweisen, die folgenden Schritte auszuführen, wenn das Computerprogrammprodukt auf der Recheneinheit ausgeführt wird: Empfangen von optischen Merkmalen von Haaren; Ermitteln von Oberflächeneigenschaften der Haare basierend auf den optischen Merkmalen; Ermitteln eines Schadens der Oberflächeneigenschaften mittels eines Mustererkennungsalgorithmus und basierend auf vorbestimmten Schäden enthaltend mindestens eine Schadensart und einen zugehörigen Schadensgrad; Ausgeben des Schadens.

Das Computerprogrammprodukt ermöglicht die Kontrolle und Nachverfolgung der Ergebnisse durch Darstellung (z.B. graphisch) der Messergebnisse im Verlauf der Zeit. Anhand der erzielten Ergebnisse gibt das Computerprogrammprodukt individuelle Behandlungs- und Produkttipps. Die Qualität der Behandlungs- und Produkttipps kann dadurch verbessert werden, dass der Benutzer zusätzlich Fragen zu seinem Haarzustand, seinen Ernährungsgewohnheiten, seinem generellen Gesundheitszustand und weiteren Verhaltensweisen beantwortet, die das Computerprogrammprodukt entsprechend verarbeiten kann. Dazu werden nicht nur z.B. Literaturdaten zu Grunde gelegt, sondern auch der Behandlungserfolg anderer Nutzer des Computerprogrammprodukts, insbesondere Behandlungserfolge anderer Nutzer, die zumindest einen ähnlichen Haarzustand aufweisen.

Die mittels des Fragebogens erfassten Daten können verwendet werden, um eine Entwicklung des Zustands der Haare des Nutzers unter den gegebenen Umständen, also den von dem Nutzer eingegebenen Daten, zu analysieren. Diese Entwicklung kann mit der Entwicklung anderer Nutzer verglichen werden. Hieraus kann gefolgert werden, ob sich während einer Behandlung mit einem bestimmten Produkt die Entwicklung von Nutzern mit ähnlichen oder gleichen Eingaben im Fragebogen gleicht oder von Nutzern mit anderen Eingaben abweicht.

So kann beispielsweise auf den Einfluss eines bestimmten Faktums auf den Erfolg der Behandlung geschlossen werden. Zeigt beispielsweise die Entwicklung einer Schadensart bei mehreren rauchenden Personen mit einem bestimmten Zigarettenkonsum (z.B. zehn Zigaretten pro Tag) eine signifikante Abweichung von der Entwicklung der gleichen Schadensart bei Nichtrauchern, kann gefolgert werden, dass Rauchen sich auf den bestimmten Parameter in einer zu beziffernden Weise auswirkt. Alternativ kann gefolgert werden, dass für Raucher ein anderes Produkt oder eine andere Behandlung empfehlenswert ist.

Die von dem Nutzer eingegebenen Daten können somit für eine globale Analyse verwendet werden, um den Erfolg einer Behandlung und die Wirksamkeit eines Produkts unter verschiedenen Bedingungen überwachen und ggf. Änderungen der Behandlung und/oder des Produkts empfehlen zu können.

Gemäß einem weiteren Aspekt ist ein Verfahren zum Bestimmen von äußeren Schäden von Haaren angegeben. Das Verfahren weist die folgenden Schritte auf: Empfangen von optischen Merkmalen von Haaren; Ermitteln von Oberflächeneigenschaften der Haare basierend auf den optischen Merkmalen; Ermitteln eines Schadens der Oberflächeneigenschaften mittels eines Mustererkennungsalgorithmus und basierend auf vorbestimmten Schäden enthaltend mindestens eine Schadensart und einen zugehörigen Schadensgrad; Ausgeben des Schadens.

Gemäß einer Ausführungsform weist das Verfahren weiter den folgenden Schritt auf: Konfigurieren des Mustererkennungsalgorithmus basierend auf einer Vielzahl von Haaren mit unterschiedlichem Schaden, wobei für jedes einzelne Haar ein Schaden abgefragt und zugeordnet wird. Dieser Schritt kann ausgeführt werden, bevor unbekannte Haarproben für eine Erkennung des äußeren Schadens der Verarbeitungseinheit zugeführt werden. Es ist aber auch denkbar, dass auf eine dedizierte Konfiguration verzichtet werden kann, beispielsweise wenn der Mustererkennungsalgorithmus schon konfiguriert ist.

Damit wird ein nicht-mechanisches, berührungsloses Verfahren zum Bestimmen des äußeren Schadens von Haaren bereitgestellt. Das Verfahren basiert auf vergrößerten optischen Abbildungen von Haarproben, welche mit Ansätzen des maschinengestützten Lernens oder Erkennens von Mustern verknüpft werden.

Gemäß einem weiteren Aspekt ist ein Verfahren zum Ermitteln eines Behandlungsmittels angegeben. Dieses Verfahren basiert auf dem gemäß obigem Verfahren bestimmten äußeren Schaden von Haaren und weist die folgenden Schritte auf: Heranziehen des bestimmten äußeren Schadens von Haaren; und Ermitteln eines Behandlungsmittels, dessen Eigenschaften dem äußeren Schaden zugeordnet sind und welches an dem äußeren Schaden eine gewünschte Wirkung entfaltet.

Das Behandlungsmittel wird in Abhängigkeit des ermittelten oder bestimmten äußeren Schadens und unter Berücksichtigung einer gewünschten Wirkung, beispielsweise gewünschter Eigenschaften der Haare nach der Behandlung, ausgesucht oder ermittelt. Bei der gewünschten Wirkung kann es sich um eine von einem Benutzer vorgegebene Wirkung oder einen gewünschten Zustand des Haars handeln. Hierbei kann es hilfreich sein, dass jedem Behandlungsmittel eine oder mehrere Schadensarten zugeordnet werden, bei der eine Anwendung möglich ist, und auch eine Wirkung, welche das Behandlungsmittel bei der entsprechenden Schadensart hat. Somit kann über einen einfachen Abgleich der Schadensart und der gewünschten Wirkung das passende Behandlungsmittel ermittelt werden.

Ausführungsbeispiele der Erfindung sind in den Figuren dargestellt und werden im Folgenden näher erläutert. Dabei zeigt:
- Fig. 1: eine schematische Darstellung einer Anordnung zum Ermitteln von äußeren Schäden von Haaren gemäß einem Ausführungsbeispiel;
- Fig. 2: eine schematische Darstellung einer Anordnung zum Ermitteln von äußeren Schäden von Haaren gemäß einem weiteren Ausführungsbeispiel;
- Fig. 3: eine schematische Darstellung einer Verarbeitungseinheit einer Anordnung gemäß einem weiteren Ausführungsbeispiel;
- Fig. 4: eine beispielhafte Aufnahme eines menschlichen Haares;
- Fig. 5: eine schematische Darstellung von Schritten eines Verfahrens gemäß einem Ausführungsbeispiel.

In der folgenden ausführlichen Beschreibung wird auf die beigefügten Zeichnungen Bezug genommen, die einen Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder funktionale oder logische Änderungen vorgenommen werden können, ohne von dem Schutzumfang der vorliegenden Erfindung abzuweichen. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

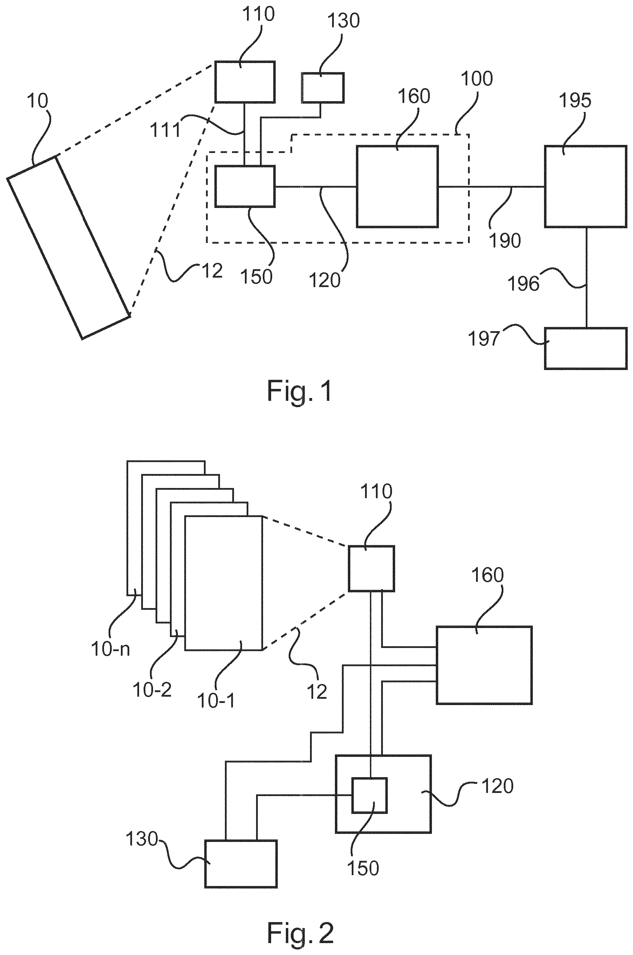

Fig. 1 zeigt eine Anordnung 100 zum Bestimmen eines äußeren Schadens einer Haarprobe 10.

Eine optische Erfassungseinheit 110 ist angeordnet, eine optische Abbildung oder Bildaufnahme der Haarprobe 10 in dem zu untersuchenden Oberflächenbereich 12 anzufertigen. Diese Bildaufnahme bildet die Grundlage für die weitere Untersuchung und wird über die Leseeinheit 150 an die Verarbeitungseinheit 160 gegeben. Die Leseeinheit 150 kann Bildaufnahmen von Haarproben auch aus einem Datenspeicher 130 auslesen.

Eine Datenübertragungsstrecke 111 verbindet die optische Erfassungseinheit 110 mit der Leseeinheit 150. Die Leseeinheit 150 kann als Schnittstelle zwischen Verarbeitungseinheit 160 und optischer Erfassungseinheit 110 ausgestaltet sein und kann beispielsweise das Datenformat der Bildaufnahmen konvertieren oder anpassen und auch eine mechanische Verbindung zwischen Verarbeitungseinheit 160 und optischer Erfassungseinheit bieten. Die Datenübertragungsstrecke 111 kann drahtlos oder drahtgebunden sein.

Die Verbindung zwischen dem Datenspeicher 130 und der Leseeinheit 150 kann ebenfalls so ausgestaltet sein, wie die Datenübertragungsstrecke 111.

Die Leseeinheit 150 liefert Eingabedaten 112 an die Verarbeitungseinheit 160. Bei den Eingabedaten 112 handelt es sich beispielsweise um Bilddaten in einem digitalen Format.

Die Verarbeitungseinheit 160 führt Analyseschritte anhand der Bilddaten der Haarprobe aus. Diese Analyseschritte können einen Abgleich mit bekannten Mustern enthalten und/oder eine Untersuchung der Bilddaten mittels künstlichen neuronalen Netzen. Diese Analyseschritte ermöglichen es, der Haarprobe einen Schaden (Schadensart und Schadensgrad) zuzuordnen, sei es durch Vergleich mit bekannten und in Schadensgruppen klassifizierten Haarproben oder mit Hilfe des künstlichen Neuronalen Netzes, welches mit bekannten und kategorisierten Haarproben konfiguriert wurde.

Die Verarbeitungseinheit 160 liefert den Schaden als Ausgabewert 190 an eine Auswerteeinheit 195. Die Auswerteeinheit 195 ermittelt ausgehend von dem Schaden der Haarprobe eine Empfehlung für die Behandlung der Haare, sei es unter Bezugnahme auf zu verwendende Behandlungsmittel (insbesondere inklusive eines Anwendungshinweises) oder ohne Behandlungsmittel. Hierzu greift die Auswerteeinheit 195 auf Informationen in einem Behandlungsmitteldatenspeicher 197 zu. Die Auswerteeinheit 195 ist mit dem Behandlungsmitteldatenspeicher 197 über ein Datennetz 196 signaltechnisch verbunden. Das Datennetz 196 kann unidirektionalen oder bidirektionalen Datenaustausch über eine drahtgebundene, drahtlose oder gemischte Verbindung ermöglichen.

Der Behandlungsmitteldatenspeicher 197 kann räumlich getrennt von der Auswerteeinheit 195 angeordnet sein kann. Beispielsweise ist der Behandlungsmitteldatenspeicher 197 so angeordnet, dass eine Vielzahl von Anordnungen 100 auf den Behandlungsmitteldatenspeicher 197 zugreifen kann. Dies ermöglicht es, dass der Behandlungsmitteldatenspeicher 197 an zentraler Stelle mit Informationen über Behandlungsmittel und/oder Behandlungshinweisen befüllt werden kann. Diese Informationen und Behandlungshinweise können unabhängig von den anfragenden Anordnungen überarbeitet und angepasst werden. Somit können jeweils aktuelle Informationen an anfragende Anordnungen 100 oder Auswerteeinheiten 195 geliefert werden.

Fig. 2 zeigt beispielhaft, wie die Verarbeitungseinheit 160 für das maschinelle Erkennen von äußeren Schäden von Haarproben vorbereitet und/oder konfiguriert wird. Eine Vielzahl von Haarproben 10-1, 10-2, ..., 10-n werden von der optischen Erfassungseinheit 110 erfasst und als Bilddaten an die Verarbeitungseinheit 160 geliefert. Jedes einzelne Bild wird auch einer Klassifizierungseinheit 120 bereitgestellt, wobei die Leseeinheit 150 als Schnittstelle zwischen optischer Erfassungseinheit 120 und Klassifizierungseinheit 120 dienen kann.

Über die Klassifizierungseinheit 120 wird, beispielsweise von einem menschlichen Bediener, jeder einzelnen Bildaufnahme einer Haarprobe ein Schaden zugeordnet. Beispielsweise werden für vorgegebene Schadensarten jeweils Schadensgrade als einzelner Wert einer Skala erfasst. Es ist aber auch denkbar, dass für eine Haarprobe nur ein einzelner Wert erfasst wird, welcher nicht nach Schadensarten unterscheidet, sondern den Gesamtzustand beschreibt.

Die Bilddaten können alternativ oder zusätzlich aus einem Datenspeicher 130 eingelesen und der Klassifizierung zugeführt werden.

Basierend auf dem gleichen Ansatz kann auch ein künstliches neuronales Netz konfiguriert werden. Dem künstlichen neuronalen Netz werden Bilddaten und zugeordnete Schäden zugeführt, womit das künstliche neuronale Netz nach der Konfiguration unbekannten Bildern einen Schaden zuordnen kann. Das neuronale Netz kann entweder einen einzelnen Wert für den Gesamtschaden ausgeben oder jeweils einen Wert für verschiedene Schadensarten, je nach Konfiguration des neuronalen Netzes.

Insbesondere ein neuronales Netz kann somit in vorteilhafter Weise dazu genutzt werden, um aus einer Bildaufnahme einer Haarprobe den äußeren Schaden zu ermitteln und anzugeben. Dies kann den Aufwand für das Bestimmen des äußeren Schadens einer Haarprobe stark reduzieren. Beispielsweise kann an einem Ort eine Bildaufnahme angefertigt und über ein Kommunikationsnetz an die Verarbeitungseinheit an einem anderen Ort übermittelt werden, wo die Haarprobe untersucht und das Ergebnis geliefert wird. Dieser Vorgang kann in wenigen Sekunden oder Minuten erledigt sein. Abgesehen von der optischen Erfassungseinheit, welche Bilder in der geforderten Vergrößerung und Auflösung erstellen können muss, ist es nicht erforderlich, die Verarbeitungseinheit räumlich zu bewegen.

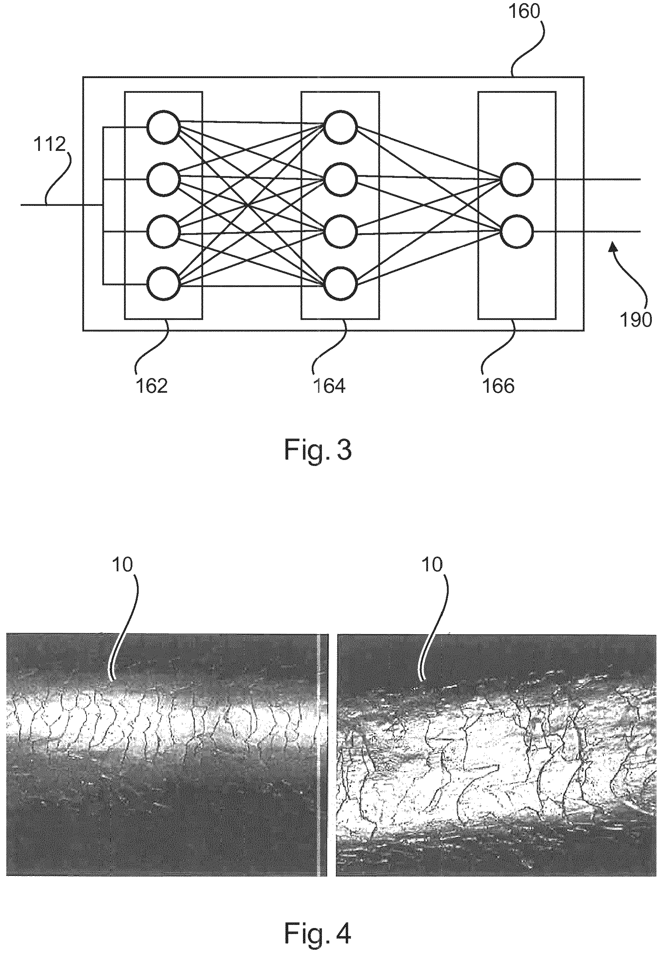

Fig. 3 zeigt eine schematische Darstellung der nicht beanspruchten Verarbeitungseinheit 160 als künstliches neuronales Netz mit einer Eingangsschicht 162, einer Zwischenschicht 164 (oder verborgene Schicht) und einer Ausgangsschicht 166.

Die Bilddaten 112 werden der Eingangsschicht 162 zugeführt. Jede Schicht enthält mehrere künstliche Neuronen, welche jeweils über mindestens eine Verbindung einen Eingabewert erhalten und ausgangsseitig mit mindestens einem Neuron der folgenden (weiter rechts liegenden) Schicht verbunden sind. Das künstliche neuronale Netz kann eine Vielzahl von Zwischenschichten 164 enthalten. Der Ausgabewert 190 kann mehrere Einzelsignale von mehreren Neuronen der Ausgabeschicht 166 enthalten, wobei beispielsweise jedes Einzelsignal einen Wert anzeigt. Beispielsweise kann ein Neuron der Ausgangsschicht einer Schadensart zugeordnet sein und sein Wert den entsprechenden Schadensgrad anzeigen.

Fig. 4 zeigt beispielhaft eine vergrößerte Bildaufnahme eines Haares, wobei die einzelnen Schuppen der Cuticula zu erkennen sind. Die linke Abbildung zeigt eine Abbildung mit gleichmäßigen Abständen der Kanten der Schuppen, wohingegen diese Abstände in der rechten Abbildung ungleichmäßig sind und auf einen äußeren Schaden des Haares hinweisen. Neben den Abständen der Kanten können andere Parameter für die Bestimmung des Schadens herangezogen werden, beispielsweise ob die Ränder rund oder ausgefranst sind, ob die Schuppen anliegen oder an ihrem Rand abstehen, die Länge der exponierten Cuticula-Zellen, das Vorkommen von Löchern und Blasen, die Oberflächenrauigkeit, Ablagerungen auf der Cuticula, oder die Sichtbarkeit der Endocuticula.

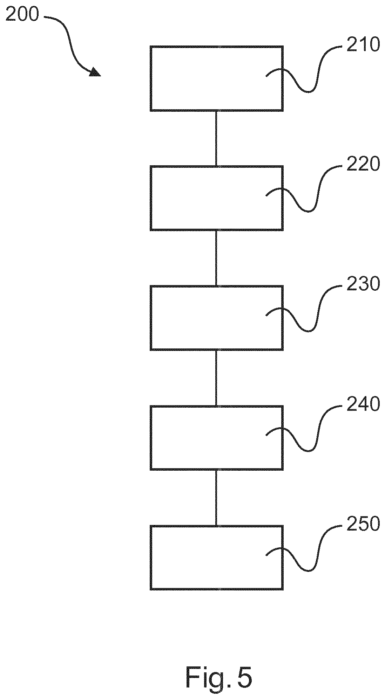

Fig. 5 zeigt eine schematische Darstellung von Verfahrensschritten eines Verfahrens 200 zum Ermitteln von äußeren Schäden eines Haares gemäß einem Ausführungsbeispiel. In einem Schritt 220 ist das Empfangen von optischen Merkmalen von Haaren vorgesehen. In einem weiteren Schritt 230 erfolgt das Ermitteln von Oberflächeneigenschaften der Haare basierend auf den optischen Merkmalen. Anschließend wird in einem folgenden Schritt 240 ein Schaden der Oberflächeneigenschaften mittels eines Mustererkennungsalgorithmus und basierend auf vorbestimmten Schäden enthaltend mindestens eine Schadensart und einen zugehörigen Schadensgrad ermittelt. In einem vierten Schritt 250 erfolgt das Ausgeben des Schadens.

Einleitend kann es Teil des Verfahrens sein, in einem Schritt 210 den Mustererkennungsalgorithmus basierend auf einer Vielzahl von Haaren mit unterschiedlichem Schaden zu konfigurieren, wobei für jedes einzelne Haar ein Schaden abgefragt und zugeordnet wird.

### LISTE DER BEZUGSZEICHEN

- 10: Analyseobjekt, Haarprobe
- 12: zu untersuchender Oberflächenbereich
- 100: Anordnung zum Bestimmen eines Schadens von Haaren
- 110: optische Erfassungseinheit
- 111: Datenübertragungsstrecke
- 112: Eingabedaten
- 120: Klassifizierungseinheit
- 130: Datenspeicher
- 150: Leseeinheit
- 160: Verarbeitungseinheit
- 162: Eingangsschicht
- 164: Zwischenschicht, verborgene Schicht
- 166: Ausgabeschicht
- 190: Ausgabewert
- 195: Auswerteeinheit
- 196: Datennetz
- 197: Behandlungsmitteldatenspeicher
- 200: Verfahren
- 210-250: Verfahrensschritte

## Patentansprüche

1. Anordnung (100) zum Bestimmen von äußeren Schäden von Haaren, die Anordnung aufweisend:
eine Leseeinheit (150), welche ausgeführt ist, optische Merkmale von Haaren in einer Bildaufnahme im sichtbaren Lichtspektrum zu erhalten, wobei die optischen Merkmale die Struktur der Cuticula umfassen;
eine Verarbeitungseinheit (160), welche ausgeführt ist, die optischen Merkmale von der Leseeinheit zu erhalten und aus den optischen Merkmalen Oberflächeneigenschaften der Haare zu ermitteln;
wobei die Verarbeitungseinheit (160) einen Mustererkennungsalgorithmus implementiert und ausgeführt ist, mittels des Mustererkennungsalgorithmus die Oberflächeneigenschaften einem vorbestimmten Schaden enthaltend mindestens eine Schadensart und einen zugehörigen Schadensgrad zuzuordnen und den Schaden auszugeben, wobei der Mustererkennungsalgorithmus die Form der Cuticula-Zellen erfasst und daraus den äußeren Schaden des Haares ermittelt indem die erkannten Oberflächeneigenschaften mit vorab bekannten und hinterlegten Schäden an Haaren abgeglichen und einem Schaden zugeordnet werden.

2. Anordnung (100) nach einem der voranstehenden Ansprüche,
wobei die Leseeinheit (150) ausgeführt ist, die optischen Merkmale von einer optischen Erfassungseinheit (110) zu erhalten.

3. Anordnung (100) nach Anspruch 2,
wobei die optische Erfassungseinheit (110) ein Mikroskop ist, welches eine mindestens 100-fache Vergrößerung aufweist, bevorzugt eine mindestens 500-fache Vergrößerung, weiter bevorzugt eine mindestens 1000-fache Vergrößerung und noch weiter bevorzugt eine mindestens 2000-fache Vergrößerung.

4. Anordnung (100) nach einem der voranstehenden Ansprüche,
wobei die Leseeinheit (150) ausgeführt ist, die optischen Merkmale aus Bildaufnahmen von Haaren zu erhalten, und/oder die Bildaufnahmen aus einem digitalen Datenspeicher (130) auszulesen.

5. Anordnung (100) nach einem der voranstehenden Ansprüche,
weiterhin aufweisend eine Auswerteeinheit (195);
wobei die Auswerteeinheit (195) ausgeführt ist, Merkmale von Behandlungsmitteln zur Behandlung von Haaren mit dem Schaden einer untersuchten Haarprobe abzugleichen und eine Wirkung der Behandlungsmittel auf die Haare unter Berücksichtigung des ermittelten Schadens zu bestimmen.

6. Anordnung (100) nach Anspruch 5,
wobei die Auswerteeinheit (195) ausgeführt ist, von einem Behandlungsmitteldatenspeicher (197) Hinweise zur Behandlung von Haaren gemäß dem ermittelten Schaden zu erhalten und die erhaltenen Hinweise auszugeben.

7. Anordnung (100) nach einem der Ansprüche 5 oder 6,
wobei die Auswerteeinheit (195) ausgeführt ist, Informationen von einem Nutzer abzufragen und diese Informationen beim Abfragen des Behandlungsmitteldatenspeichers (197) zusätzlich zu berücksichtigen, um von dem Behandlungsmitteldatenspeicher (197) Merkmale von Behandlungsmitteln zur Behandlung von Haaren gemäß den abgefragten Informationen zu erhalten.

8. Anordnung (100) nach einem der Ansprüche 5 bis 7,
wobei die Auswerteeinheit (195) ausgeführt ist, Informationen über ein Behandlungsmittel, z.B. eine Produktbezeichnung, Informationen betreffend Inhaltsstoffe und/oder Zusammensetzung eines Behandlungsmittels und/oder Anwendungshinweise zur Behandlung von Haaren auszugeben.

9. Computerprogrammprodukt, welches ausgeführt ist, auf einer Recheneinheit ausgeführt zu werden und einen Prozessor der Recheneinheit anzuweisen, die folgenden Schritte auszuführen, wenn das Computerprogrammprodukt auf der Recheneinheit ausgeführt wird:
Empfangen von optischen Merkmalen von Haaren in einer Bildaufnahme im sichtbaren Lichtspektrum, wobei die optischen Merkmale die Struktur der Cuticula umfassen;
Ermitteln von Oberflächeneigenschaften der Haare basierend auf den optischen Merkmalen;
Ermitteln eines Schadens der Oberflächeneigenschaften mittels eines Mustererkennungsalgorithmus und basierend auf vorbestimmten Schäden enthaltend mindestens eine Schadensart und einen zugehörigen Schadensgrad, wobei der Mustererkennungsalgorithmus die Form der Cuticula-Zellen erfasst und daraus den äußeren Schaden des Haares ermittelt, indem die erkannten Oberflächeneigenschaften mit vorab bekannten und hinterlegten Schäden an Haaren abgeglichen und einem Schaden zugeordnet werden;
Ausgeben des Schadens.

10. Verfahren (200) zum Bestimmen von äußeren Schäden von Haaren, aufweisend die folgenden Schritte:
Empfangen (220) von optischen Merkmalen von Haaren in einer Bildaufnahme im sichtbaren Lichtspektrum, wobei die optischen Merkmale die Struktur der Cuticula umfassen;
Ermitteln (230) von Oberflächeneigenschaften der Haare basierend auf den optischen Merkmalen;
Ermitteln (240) eines Schadens der Oberflächeneigenschaften mittels eines Mustererkennungsalgorithmus und basierend auf vorbestimmten Schäden enthaltend mindestens eine Schadensart und einen zugehörigen Schadensgrad, wobei der Mustererkennungsalgorithmus die Form der Cuticula-Zellen erfasst und daraus den äußeren Schaden des Haares ermittelt, indem die erkannten Oberflächeneigenschaften mit vorab bekannten und hinterlegten Schäden an Haaren abgeglichen und einem Schaden zugeordnet werden;
Ausgeben (250) des Schadens.

11. Verfahren nach Anspruch 10,
weiter aufweisend den Schritt:
Konfigurieren (210) des Mustererkennungsalgorithmus basierend auf einer Vielzahl von Haaren mit unterschiedlichem Schaden, wobei für jedes einzelne Haar ein Schaden abgefragt und zugeordnet wird.

12. Verfahren zum Ermitteln eines Behandlungsmittels basierend auf dem in Anspruch 10 oder 11 bestimmten äußeren Schaden von Haaren, aufweisend die folgenden Schritte:
Ausführen des Verfahrens aus Anspruch 10 oder Anspruch 11; Heranziehen des bestimmten äußeren Schadens von Haaren;
Ermitteln eines Behandlungsmittels, dessen Eigenschaften dem äußeren Schaden zugeordnet sind und welches an dem äußeren Schaden eine gewünschte Wirkung entfaltet.

## Claims

1. An apparatus (100) for determining external damage to hair, the apparatus comprising:
a reading unit (150) configured to obtain optical characteristics of hair in an image captured in the visible light spectrum, wherein the optical characteristics comprise the structure of the cuticle;
a processing unit (160) configured to receive the optical features from the reading unit and to determine surface properties of the hair from the optical features;
wherein the processing unit (160) implements a pattern recognition algorithm and is configured to use the pattern recognition algorithm to assign the surface properties to at least one type of damage comprising a predetermined damage, and an associated degree of damage, and to output the damage, wherein the pattern recognition algorithm detects the shape of the cuticle cells and determines the external damage to the hair therefrom by comparing the detected surface properties with previously known and stored hair damage and assigning them to a specific type of damage.

2. An apparatus (100) according to one of the preceding claims,
wherein the reading unit (150) is configured to obtain the optical features from an optical detection unit (110).

3. An apparatus (100) according to claim 2,
wherein the optical detection unit (110) is a microscope having a magnification of at least 100×, preferably at least 500×, more pre ly at least 1000×, and even more pre ly at least 2000×.

4. An apparatus (100) according to one of the preceding claims,
wherein the reading unit (150) is configured to obtain the optical features from images of hair and/or to read the images from a digital data storage (130).

5. An apparatus (100) according to one of the preceding claims,
further comprising an evaluation unit (195);
wherein the evaluation unit (195) is configured to compare characteristics of treatment agents for treating hair with the damage of an examined hair sample and to determine an effect of the treatment agents on the hair, taking into account the determined damage.

6. An apparatus (100) according to claim 5,
wherein the evaluation unit (195) is configured to receive instructions from a treatment agent data store (197) for treating hair in accordance with the determined damage and to output the received instructions.

7. An apparatus (100) according to one of claims 5 or 6,
wherein the evaluation unit (195) is configured to request information from a user and to additionally take this information into account when querying the treatment agent data store (197) in order to obtain from the treatment agent data store (197) characteristics of treatment agents for treating hair in accordance with the requested information.

8. An apparatus (100) according to any one of claims 5 to 7,
wherein the evaluation unit (195) is configured to output information about a treatment agent, e.g. a product name, information concerning ingredients and/or the composition of a treatment agent and/or instructions for use for the treatment of hair.

9. A computer program product configured to be executed on a computing unit and to instruct a processor of the computing unit to perform the following steps when the computer program product is executed on the computing unit:
receiving optical characteristics of hair in an image capture in the visible light spectrum, wherein the optical characteristics comprise the structure of the cuticle;
determining surface properties of the hair based on the optical characteristics;
determining damage to the surface properties by means of a pattern recognition algorithm and based on predetermined damage types comprising at least one type of damage and an associated degree of damage, wherein the pattern recognition algorithm detects the shape of the cuticle cells and determines the external damage to the hair by comparing the detected surface properties with pre-known and stored hair damage and assigning them to a specific type of damage;
Outputting the damage.

10. A method (200) for determining external damage to hair, comprising the following steps:
Receiving (220) optical characteristics of hair in an image captured in the visible light spectrum, wherein the optical characteristics include the structure of the cuticle;
determining (230) surface properties of the hair based on the optical characteristics;
determining (240) damage to the surface properties by means of a pattern recognition algorithm and based on predetermined damage types comprising at least one type of damage and an associated degree of damage, wherein the pattern recognition algorithm detects the shape of the cuticle cells and determines the external damage to the hair by comparing the detected surface properties with pre-known and stored hair damage and assigning them to a specific type of damage;
Outputting (250) the damage.

11. The method according to claim 10,
further comprising the step of:
Configuring (210) the pattern recognition algorithm based on a plurality of hairs with varying damage, wherein damage is queried and assigned for each individual hair.

12. A method for determining a treatment agent based on the external damage to hair determined in claim 10 or 11, comprising the following steps:
referring to the determined external damage to hair;
Determining a treatment agent whose properties correspond to the external damage and which exerts a desired effect on the external damage.

## Revendications

1. Dispositif (100) destiné à déterminer les dommages externes des cheveux, le dispositif comprenant :
une unité de lecture (150) conçue pour obtenir des caractéristiques optiques des cheveux dans une image enregistrée dans le spectre de la lumière visible, lesdites caractéristiques optiques comprenant la structure de la cuticule ;
une unité de traitement (160) conçue pour recevoir les caractéristiques optiques provenant de l'unité de lecture et pour déterminer, à partir de ces caractéristiques optiques, les propriétés de surface des cheveux ;
l'unité de traitement (160) mettant en œuvre un algorithme de reconnaissance de formes et étant conçue pour attribuer, à l'aide de cet algorithme, les propriétés de surface à un dommage prédéterminé comprenant au moins un type de dommage et un degré de dommage associé, et pour signaler le dommage, l'algorithme de reconnaissance de formes détectant la forme des cellules de la cuticule et déterminant à partir de celle-ci le dommage externe du cheveu en comparant les propriétés de surface détectées avec des dommages aux cheveux connus et enregistrés au préalable, puis en les attribuant à un dommage.

2. Dispositif (100) selon l'une des revendications précédentes,
dans lequel l'unité de lecture (150) est conçue pour recevoir les caractéristiques optiques d'une unité de détection optique (110).

3. Dispositif (100) selon la revendication 2,
dans lequel l'unité de détection optique (110) est un microscope présentant un grossissement d'au moins 100 fois, de préférence d'au moins 500 fois, de manière encore plus préférentielle d'au moins 1 000 fois et de manière encore plus préférentielle d'au moins 2 000 fois.

4. Dispositif (100) selon l'une des revendications précédentes,
dans lequel l'unité de lecture (150) est conçue pour obtenir les caractéristiques optiques à partir d'images de cheveux et/ou pour lire les images à partir d'une mémoire de données numérique (130).

5. Dispositif (100) selon l'une des revendications précédentes,
comprenant en outre une unité d'évaluation (195) ;
l'unité d'évaluation (195) étant conçue pour comparer les caractéristiques de produits de traitement destinés au traitement de cheveux présentant les dommages d'un échantillon de cheveux examiné et pour déterminer un effet des produits de traitement sur les cheveux en tenant compte des dommages constatés.

6. Dispositif (100) selon la revendication 5,
dans lequel l'unité d'évaluation (195) est conçue pour recevoir, à partir d'une mémoire de données de produits de traitement (197), des indications relatives au traitement des cheveux en fonction des dommages constatés et pour fournir les indications reçues.

7. Dispositif (100) selon l'une des revendications 5 ou 6,
dans lequel l'unité d'évaluation (195) est conçue pour demander des informations à un utilisateur et pour prendre en compte ces informations en plus lors de l'interrogation de la mémoire de données de produits de traitement (197), afin d'obtenir de la mémoire de données de produits de traitement (197) des caractéristiques de produits de traitement pour le traitement des cheveux en fonction des informations demandées.

8. Dispositif (100) selon l'une des revendications 5 à 7,
dans lequel l'unité d'évaluation (195) est conçue pour fournir des informations sur un produit de soin, par exemple une désignation de produit, des informations concernant les ingrédients et/ou la composition d'un produit de soin et/ou des conseils d'utilisation pour le traitement des cheveux.

9. Produit logiciel conçu pour être exécuté sur une unité de calcul et pour ordonner à un processeur de l'unité de calcul d'exécuter les étapes suivantes lorsque le produit logiciel est exécuté sur l'unité de calcul :
Recevoir des caractéristiques optiques des cheveux dans une image enregistrée dans le spectre de la lumière visible, lesdites caractéristiques optiques comprenant la structure de la cuticule ;
déterminer les propriétés de surface des cheveux sur la base des caractéristiques optiques ;
déterminer un dommage des propriétés de surface à l'aide d'un algorithme de reconnaissance de formes et sur la base de dommages prédéterminés comprenant au moins un type de dommage et un degré de dommage associé, l'algorithme de reconnaissance de formes détectant la forme des cellules de la cuticule et déterminant à partir de celle-ci les dommages externes du cheveu en comparant les propriétés de surface détectées avec des dommages aux cheveux connus et enregistrés au préalable, puis en les attribuant à un dommage ;
Affichage du dommage.

10. Procédé (200) pour déterminer les dommages externes des cheveux, comprenant les étapes suivantes :
Recevoir (220) des caractéristiques optiques des cheveux dans une image prise dans le spectre de la lumière visible, lesdites caractéristiques optiques comprenant la structure de la cuticule ;
déterminer (230) les propriétés de surface des cheveux sur la base des caractéristiques optiques ;
Détermination (240) d'un dommage affectant les propriétés de surface à l'aide d'un algorithme de reconnaissance de formes et sur la base de dommages prédéfinis comprenant au moins un type de dommage et un degré de dommage associé, l'algorithme de reconnaissance de formes détectant la forme des cellules de la cuticule et déterminant à partir de celle-ci l'endommagement externe du cheveu en comparant les caractéristiques de surface détectées avec des endommagements des cheveux connus et enregistrés au préalable, puis en les attribuant à un endommagement ;
Affichage (250) du dommage.

11. Procédé selon la revendication 10,
comprenant en outre l'étape suivante :
configurer (210) l'algorithme de reconnaissance de formes sur la base d'une pluralité de cheveux présentant des dommages différents, un dommage étant interrogé et attribué pour chaque cheveu individuel.

12. Procédé pour déterminer un produit de traitement sur la base des dommages externes des cheveux déterminés selon la revendication 10 ou 11, comprenant les étapes suivantes :
prise en compte des dommages externes déterminés des cheveux ;
déterminer un produit de traitement dont les propriétés correspondent aux dommages externes et qui produit un effet souhaité sur les dommages externes.
